# EUROPEAN PATENT APPLICATION

(11) **EP 2 388 331 A1**
(43) Date of publication of application: **23.11.2011**
(21) Application number: 10163662.9
(22) Date of filing: 21.05.2010
(51) Int. Cl.: C12N 15/80

(54) **Constitutive promoter**

(71) Applicant: Nederlandse Organisatie voor Toegepast -Natuurwetenschappelijk Onderzoek TNO, 2628 VK Delft (NL)
(72) Inventor: The designation of the inventor has not yet been filed
(74) Representative: Hatzmann, Martin

(57) **Abstract**

The invention relates to a new, strong and constitutive promoter derived from the *Aspergillus niger* hemolysin gene, comprising the sequence 1-1130 of the sequence depicted in SEQ ID NO: 1 or a truncated variant thereof that has promoter activity, or a sequence that is more than 70% identical therewith. Preferably such a promoter with high identity is chosen from the group of orthologous promoters, comprising the promoter of the genes encoded by A0090010000018 from A. oryzae (SEQ ID NO:2), Pc12g15350 from *P. chrysogenum* (SEQ ID NO:3), ATEG_03556 from A. *terreus* (SEQ ID NO:4) and AN1553.2 from *A*. *nidulans* (SEQ ID NO:5). Also part of the invention are constructs with such a promoter, operably linked with a sequence that needs to be expressed and host cells comprising such constructs. Further methods of using this promoter to drive expression of a nucleotide sequence that is operably linked therewith are disclosed.

## Description

### FIELD OF THE INVENTION

The invention relates to the field of genetic engineering, more specifically to expression of proteins with recombinant technology. In particular, the invention relates to gene expression in fungi, especially *Aspergillus* by placing these genes behind a strong constitutive promoter.

### BACKGROUND

Molecular biotechnology is a discipline that is based on the ability of researchers to transfer specific units of genetic information from one organism to another with the goal of producing commercially relevant amounts of useful products. One of the goals of this cloning process is to achieve maximum expression of the cloned gene. Recombinant production of a product encoded by a gene is accomplished by constructing expression vectors suitable for use in a host cell in which the nucleic acids coding for a desired product is placed under the expression control of a promoter. The expression vector is introduced into a host cell by various techniques, such as transformation, and production of the desired product is then achieved by culturing the transformed host cell under suitable conditions necessary for the functioning of the promoter included in the expression vector.

Genes encoding proteins contain promoter regions of DNA which are essentially attached to the 5' terminus of the protein coding region. The promoter regions contain the binding site for RNA polymerase II. RNA polymerase II effectively catalyses the assembly of the messenger RNA complementary to the appropriate DNA strand of the coding region. Other features important or essential to the proper functioning and control of the coding region are also contained in the promoter region, upstream of the start of the coding region.

Filamentous fungi, particularly the filamentous ascomycetes such as *Aspergillus,* e.g. *Aspergillus niger,* represent a class of micro-organisms suitable as recipients of foreign genes coding for valuable proteins. *Aspergillus niger* and related species are currently used widely in the industrial production of enzymes e.g. for use in the food industry. Their use is based on the secretory capacity of the microorganism. Because they are well characterized and because of their wide use and acceptance, there is both industrial and scientific incentive to provide genetically modified and enhanced cells of A. *niger* and related species including A. *nidulans, A. oryzae, A.sojae* in order to obtain useful proteins.

Numerous promoters are already known to be functional in various host cells. There are examples of cross-species use of promoters in fungal host cells: the promoter of the *Aspergillus nidulans* gpdA gene is known to be functional in *Aspergillus niger* (Punt P J, et al., 1991, J. Biotechnol. January; 17(1):19-33) and many other filamentous fungi (Meyer et al., 2010, in "Manual of Industrial Microbiology and Biotechnology", Richard H. Baltz (Editor), Julian E. Davies (Ed.), Arnold L. Demain,(Ed.) Vol. 3 ASM press, Washington DC. pp 318-329

Another example is the A. *niger* beta-xylosidase xlnD promoter used in *A. niger* and A. *nidulans* (Transcriptional regulation of the xylanolytic enzyme system of Aspergillus, van Peij, NNME, 1999, PhD-thesis Landbouwuniversiteit Wageningen, the Netherlands, ISBN 90-5808-154-0) and the expression of the *Escherichia coli* beta-glucuronidase gene in A. *niger, A. nidulans* and *Cladosporium fulvum* as described in Roberts, I.N. et al., 1989, Curr Genet. 15(3):177-80.

However, there is still a need for improved promoters for controlling the expression of introduced genes, for controlling the level of expression of endogenous genes, for controlling the regulation of expression of endogenous genes or for mediating the inactivation of an endogenous gene, or for producing polypeptides, or for combination of the previous applications. These improved promoters may for example be stronger than the previous known ones. They may also be inducible by a specific convenient substrate or compound. Knowing several functional promoters is also an advantage when one envisages to simultaneously over express various genes in a single host. To prevent squelching (titration of specific transcription factors), it is preferable to use multiple distinct promoters, one specific promoter for each gene to be expressed.

While numerous promoters are known in the art, there is a need for new promoters, which control the expression of heterologous genes and coding sequences. This invention describes new promoters used to express genes in host cells, such as bacterial and fungal cells.

### SUMMARY OF THE INVENTION

The present inventors now have found a new, strong constitutive promoter derived from the *Aspergillus niger* hemolysin gene, comprising the sequence 1-1130 of the sequence depicted in SEQ ID NO: 1 or a truncated variant thereof that has promoter activity, or a sequence that is more than 70% identical therewith, preferably more than 80% identical therewith, more preferably more than 85%, more preferably more than 90%, more preferably more than 95%, more preferably more than 96%, 97%, 98%, or 99% identical therewith. Said homologous promoter sequence is preferably chosen from the group of orthologous promoters, comprising the promoter of the gene encoded by A0090010000018 from A. oryzae (SEQ ID NO:2), the promoter of the gene encoded by Pc12g15350 from *P. chrysogenum* (SEQ ID NO:3), the promoter of the gene encoded by ATEG_03556 from A. *terreus* (SEQ ID NO:4) and the promoter of the gene encoded by AN1553.2 from A. *nidulans* (SEQ ID NO:5).

The invention further comprises a DNA construct comprising a promoter DNA sequence according to the invention and a coding sequence in operative association with said promoter DNA sequence such that the coding sequence can be expressed under the control of the promoter DNA sequence.

Also encompassed in the invention is a host cell, preferably a fungal host cell, comprising said DNA construct. More specifically, said host cell is a cell from the genus Aspergillus, Penicillium, Chrysosporium, Trichoderma or Neurospora more preferably a host cell from an Aspergillus niger, Aspergillus sojae, Aspergillus oryzae, Chrysosporium lucknowense, Trichoderma reesei, or Penicillium chrysogenum species.

The invention further comprises a method for expression of a coding sequence in a suitable host cell comprising:
(a) providing a DNA construct according to the invention,
(b) transforming a suitable host cell with said DNA construct, and
(c) culturing the suitable host cell under culture conditions conducive to expression of the coding sequence.

The invention also relates to a method for the production of a biological compound in a suitable host cell comprising:
(a) providing a DNA construct according to the invention,
(b) transforming a suitable host cell with said DNA construct, and
(c) culturing the suitable host cell under culture conditions conducive to expression of the coding sequence, and optionally
(d) recovering the biological compound from the culture broth.

More specifically in such a method the biological compound produced is a polypeptide, preferably wherein the polypeptide produced is encoded by the coding sequence present in the DNA construct as defined above. Alternatively in such a method the biological compound produced is a metabolite.

Further preferably in such a method the coding sequence present in the DNA construct encodes an enzyme involved in the production of the metabolite.

In a further embodiment, the invention comprises the use of a promoter according to the invention for the production of a biological compound in a suitable host cell.

### LEGENDS TO THE FIGURES

Figure 1: Phylogenetic tree of the identified hemolysin genes
Figure 2: Conserved regions in the promoter regions of the most similar hemolysin genes. The ATG in this case is at position 1100, so the upstream region in front of ATG is shown. The first marked region is around -400bp upstream of the ATG and the second region is around -300pb.

### DETAILED DESCRIPTION

"Recombinant DNA technology" refers to procedures used to join together DNA sequences as described, for example, in Sambrook et al., 1989, Cold Spring Harbor, NY: Cold Spring Harbor Laboratory Press.

"Coding sequence" refers to a nucleotide (DNA or RNA) sequence that codes for a specific amino acid sequence and excludes the non-coding sequences. It may constitute an "uninterrupted coding sequence", i. e., lacking an intron, such as in a cDNA or it may include one or more introns bound by appropriate splice junctions. An "intron" is a sequence of RNA which is contained in the primary transcript but which is removed through cleavage and re-ligation of the RNA within the cell to create the mature mRNA that can be translated into a protein. Also the DNA coding for said sequence of RNA is designated as "intron". "Exons" are the coding parts of the DNA or RNA sequence, which are separated from each other by introns.

"Expression" refers to the transcription and/or translation of an endogenous gene or a transgene. In the case of antisense constructs, for example, expression may refer to the transcription of the antisense DNA only.

"Polynucleotide construct" or "DNA construct" as used herein means a nucleotide sequence (DNA sequence) capable of directing expression of a particular nucleotide sequence in an appropriate host cell, comprising a promoter operably linked to the nucleotide sequence of interest which is optionally operably linked to 3' sequences, such as 3' regulatory sequences or termination signals. It also typically comprises sequences required for proper translation of the nucleotide sequence. The coding region usually codes for a protein of interest but may also code for a functional RNA of interest, for example antisense RNA or a nontranslated RNA that, in the sense or antisense direction or as a double-stranded sequence, inhibits expression of a particular gene, e. g. through antisense inhibition or RNA interference (RNAi). The polynucleotide construct comprising the nucleotide sequence of interest may be chimeric, meaning that the nucleotide sequence is comprised of more than one DNA sequences of distinct origin which are fused together by recombinant DNA techniques resulting in a nucleotide sequence which does not occur naturally, and which particularly does not occur in the host cell to be transformed. The polynucleotide construct may also be one which is naturally occurring but has been obtained in a recombinant form useful for heterologous expression. Typically, however, the polynucleotide construct is heterologous with respect to the host, i.e., the particular DNA sequence of the polynucleotide construct does not occur naturally in the host cell and must have been introduced into the host cell or an ancestor of the host cell by a transformation event.

"Gene" refers to a coding sequence and associated regulatory sequences wherein the coding sequence is transcribed into RNA such as mRNA, rRNA, tRNA, snRNA, sense RNA or antisense RNA. Examples of regulatory sequences are promoter sequences, 5' and 3' untranslated sequences and termination sequences. Further elements that may be present are, for example, introns.

"Heterologous" as used herein means of different natural or of synthetic origin. For example, if a host cell is transformed with a nucleic acid sequence that does not occur in the untransformed host cell, that nucleic acid sequence is said to be heterologous with respect to the host cell. The transforming nucleic acid may comprise a heterologous promoter, heterologous coding sequence, or heterologous termination sequence.

Alternatively, the nucleic acid which is introduced into a host cell may be completely heterologous or may comprise any possible combination of heterologous and endogenous nucleic acid sequences. Similarly, heterologous refers to a nucleotide sequence derived from and inserted into the same natural, original cell type, but which is present in a non-natural state, e. g. a different copy number, or under the control of different regulatory elements.

A regulatory DNA sequence is said to be "operably linked to" or " in operative association with" a DNA sequence that codes for an RNA or a protein if the two sequences are situated such that the regulatory DNA sequence affects expression of the coding DNA sequence.

"Regulatory elements" refer to sequences involved in conferring the expression of a nucleotide sequence. Regulator elements comprise a promoter operably linked to the nucleotide sequence of interest and, optionally, 3' sequences, such as 3' regulatory sequences or termination signals. They also typically encompass sequences required for proper translation of the nucleotide sequence.

"Promoter" refers to a nucleotide sequence, usually upstream (5') to its coding sequence, which controls the expression of the coding sequence by providing the recognition for RNA polymerase and other factors required for proper transcription. The promoter of the invention includes a minimal promoter that is a short DNA sequence comprised of a TATA box and other sequences that serve to specify the site of transcription initiation, to which regulatory elements are added for control of expression. It also comprises regulatory elements that are capable of controlling the expression of a coding sequence or functional RNA. Such sequences consist of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue specificity of a promoter. It is capable of operating in both orientations (normal or flipped), and is capable of functioning even when moved either upstream or downstream from the promoter. Both enhancers and other upstream promoter elements bind sequence-specific DNA-binding proteins that mediate their effects. Promoters generally may be derived in their entirety from a native gene, or be composed of different elements derived from different promoters found in nature, or even be comprised of synthetic DNA segments. A promoter may also contain DNA sequences that are involved in the binding of protein factors which control the effectiveness of transcription initiation in response to physiological or developmental conditions.

The "initiation site" is the position surrounding the first nucleotide that is part of the transcribed sequence, which is also defined as position +1. With respect to this site all other sequences of the gene and its controlling regions are numbered. Downstream sequences (i. e., further protein encoding sequences in the 3' direction) are denominated positive, while upstream sequences (mostly of the controlling regions in the 5'direction) are denominated negative.

"Constitutive expression" refers to expression using a constitutive promoter.

"Constitutive promoter" refers to a promoter that is able to express the open reading frame (ORF) that it controls in all or nearly all of the host cells during all or nearly all developmental stages of the host.

The terms "open reading frame" and "ORF" refer to the amino acid sequence encoded between translation initiation and termination codons of a coding sequence. The terms "initiation codon" and "termination codon" refer to a unit of three adjacent nucleotides ('codon') in a coding sequence that specifies initiation and chain termination, respectively, of protein synthesis (mRNA translation).

"Overexpression" or "overproduction" refers to a level of expression or production of a protein or metabolite which exceeds the demand and may lead to accumulation and storage.

The term "vector" as used herein, includes reference to an autosomal expression vector and to an integration vector used for integration into the chromosome.

The term "expression vector" refers to a DNA molecule, linear or circular, that comprises a segment encoding a polypeptide of interest under the control of (i.e., operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a fungal-recognized transcription and translation initiation region, (b) a coding sequence for a polypeptide of interest, and (c) a fungal-recognized transcription and translation termination region.

"Plasmid" refers to autonomously replicating extrachromosomal DNA which is not integrated into a microorganism's genome and is usually circular in nature. An "integration vector" refers to a DNA molecule, linear or circular, that can be incorporated in a microorganism's genome and provides for stable inheritance of a gene encoding a polypeptide of interest. The integration vector generally comprises one or more segments comprising a gene sequence encoding a polypeptide of interest under the control of (i.e., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the host cell, but which has a replicon which is nonfunctional in that organism. Integration of the segment comprising the gene of interest may be selected if an appropriate marker is included within that segment.

"Transformation" and "transforming", as used herein, refers to the insertion of an exogenous polynucleotide into a host cell, irrespective of the method used for the insertion, for example, direct uptake, transduction, mating or electroporation. The exogenous polynucleotide may be maintained as a non-integrated vector, for example, a plasmid, or alternatively, may be integrated into the host cell genome.

By "host cell" is meant a cell which contains a vector or recombinant nucleic acid molecule and supports the replication and/or expression of the vector or recombinant nucleic acid molecule. Host cells may be prokaryotic cells such as E. coli, or eukaryotic cells such as yeast, fungus, plant, insect, amphibian, or mammalian cells. Preferably, host cells are fungal cells.

The term "fungus" or "fungi" includes a wide variety of nucleated, spore-bearing organisms which are devoid of chlorophyll. Examples of fungi include yeasts, mildews, molds, rusts and mushrooms. Preferred fungi in aspects of the present invention are organisms of the genera Aspergillus, Neurospora, Sclerotina, Gibberella, Coniothyrium, Psiticum, Myceliophthora, Corynascus, Thielavia, Acremonium, Magnaporthe, Podospora, Chaetomium, Phaeosphaeria, Botryotinia, Neosartorya, Pyrenophora, Panicum, Aureococcus Penicillium and Chrysospsorium.

The terms "isolated" or "purified" as used herein refer to a nucleic acid or protein or peptide that is removed from at least one component with which it is naturally associated. In the present invention, an isolated nucleic acid can include a vector comprising the nucleic acid. Purified as used herein to describe a polypeptide produced by cultivation of a recombinant host cell refers to removing that polypeptide from at least one component with which it is naturally associated in the host cell or culture medium.

It was found by the inventors that the promoter of the hemolysin gene, identified as An19g00210, discovered from *A*. *niger,* is a very strong constitutive promoter which can be used in recombinant technology for expression of biological substances in fungi. This promoter has a sequence as illustrated in SEQ ID NO: 1 (nucleotides 1 - 1130), which can be used to be put before an open reading frame encoding a gene of interest.

In analogy with other known fungal promoters, the essential characteristics of the promoter will reside in those parts of the promoter that are close to the initiation site, meaning that a truncated promoter, i.e. a promoter that is shortened at the 3' side will also be functional. A truncated promoter according to the present invention will consist of at least about 200 nucleotides (nucleotides 930-1130 of SEQ ID NO:1), more preferably at least 400 nucleotides (nucleotides 730-1130 of SEQ ID NO:1), more preferably at least 600 nucleotides (nucleotides 530-1130 of SEQ ID NO:1), more preferably at least 800 nucleotides (nucleotides 330-1130 of SEQ ID NO:1), most preferably at least 1000 nucleotides (nucleotides 130-1130 of SEQ ID NO:1). The truncated promoter of the invention will also have the same functionality as the full length promoter, although the level of expression may be somewhat less than with the full length promoter.

Also part of the invention are promoters with a large sequence identity with the promoter of the invention and which also have the same functionality as the promoter of the invention, i.e. showing promoter activity when operably linked with an open reading frame encoding a sequence to be expressed in a host cell. The percentage identity is at least 70% and preferably at least 75%, more preferably at least 80%, more preferably at least 85%, more preferably at least 90%, more preferably at least 95%, 96%, 97%, 98% or 99%.

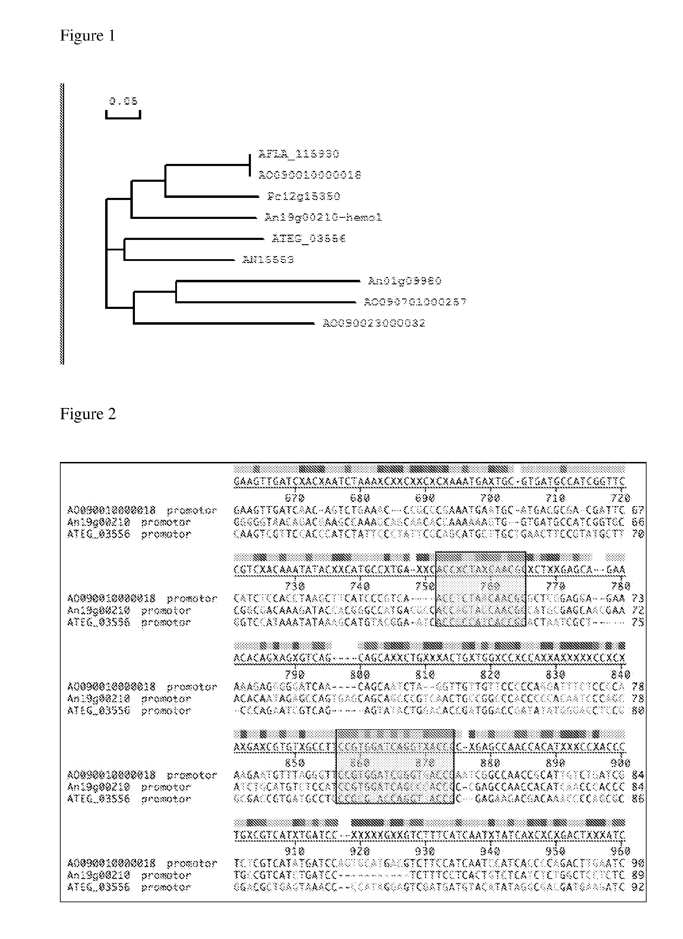

The promoter of the present invention is the promoter for the hemolysin gene of A. niger. It is to be expected that the promoter for the orthologous genes in other organisms, especially fungal organisms, more particularly other Aspergillus species, will also have similar charachteristics as the promoter of the current invention. Therefore, also the promoters of the orthologous hemolysing genes are included in the present invention. "Orthologous hemolysin genes", as used herein, are genes from a disparate species, so similar in genetic sequence that it is assumed to have originated from a single gene in a common ancestral species. Orthologous genes have been found in A. *oryzae* (AO090010000018, A0090701000257 and A0090023000032), *A*. *terreus* (ATEG_035556) and *Trichoderma reesii* (basepairs 6654-6331 in the genomic sequence). Especially A0090010000018 and ATEG_035556 have a relatively high homology with the *A*. *niger* hemolysin promoter, especially in two conserved boxes that are approximately 300 and 400 bases upstream of the coding sequence (see Fig. 2).

As is shown in the Examples, the promoter of the present invention is able to drive expression of a heterologous gene when this gene is placed under control of the promoter. Thus, the present invention also covers constructs wherein the promoter of the invention is operably linked with a heterologous DNA sequence. These constructs will preferably be transformation or expression vectors.

The expression vectors fall into two classes, integrating plasmids and autonomously replicating plasmids. To demonstrate the utility of these vectors for gene expression has been dramatically facilitated by including a reporter gene that can be assayed easily. One example of such a reporter is the ß-galactosidase coding LacZ gene from *E*. *coli.* The encoded ß-galactosidase is easily assayed, making it ideal for assessing gene expression in *A. niger.*

In another embodiment, the promoter of the invention can be used to (over)express natural substances, such as proteins or RNA in a host cell.. Overexpression of these natural substances can be effected in several ways. It can be caused by transforming a host cell with a gene coding for a protein or a sequence producing an mRNA.

Host cells used in the invention are preferably cells of filamentous fungi, yeasts and/or bacteria, such as, but not limited to, Aspergillus sp., such as the fungi A. terreus, A. itaconicus and A. niger, Aspergillus nidulans, Aspergillus oryzae, Aspergillus sojae or Aspergillus fumigatus, Trichoderma reesei, Hypocrea jecorina, Penicillium chrysosporium, Ustilago zeae, Ustilago maydis, Ustilago sp., Candida sp., Yarrowia lipolytica, Rhodotorula sp. and Pseudozyma antarctica, the bacterium E. coli and the yeast Saccharomyces cerevisiae. Recombinant host cells described above can be obtained using methods known in the art for providing cells with recombinant nucleic acids. These include transformation, transconjugation, transfection or electroporation of a host cell with a suitable plasmid (also referred to as vector) comprising the nucleic acid construct of interest operationally coupled to the promoter sequence of the invention to drive expression. Host cells of the invention are preferably transformed with a nucleic acid construct as further defined below and may comprise a single but preferably comprises multiple copies of the nucleic acid construct. The nucleic acid construct may be maintained episomally and thus comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Preferably, however, the nucleic acid construct is integrated in one or more copies into the genome of the host cell. Integration into the host cell's genome may occur at random by illegitimate recombination but preferably the nucleic acid construct is integrated into the host cell's genome by homologous recombination as is well known in the art of fungal molecular genetics (see e.g. WO 90/14423, EP-A-0 481 008, EP-A-0 635 574, US 6,265,186 and Gouka et al, 1995). Most preferably for homologous recombination the ku70D/ku80D technique is used as described for instance in WO 02/052026.

Transformation of host cells with the nucleic acid constructs of the invention and additional genetic modification of the fungal host cells of the invention as described above may be carried out by methods well known in the art. Such methods are e.g. known from standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987). Methods for transformation and genetic modification of fungal host cells are known from e.g. EP-A-0 635 574, 15 WO 98/46772, WO 99/60102 and WO 00/37671.

In another aspect the invention relates to a vector comprising a nucleotide sequence encoding a biologically active molecule, such as a protein or an mRNA, as defined above and usable for transformation of a host cell as defined above. In the nucleic acid construct, the coding nucleotide sequences is/are operably linked to the promoter of the invention for control and initiation of transcription of the nucleotide sequence in the host cell

In the nucleic acid construct, the 3'-end of coding nucleotide acid sequence(s) preferably is/are operably linked to a transcription terminator sequence. Preferably the terminator sequence is operable in a host cell of choice. In any case the choice of the terminator is not critical; it may e.g. be from any fungal gene, although terminators may sometimes work if from a non-fungal, eukaryotic, gene. The transcription termination sequence further preferably comprises a polyadenylation signal.

Optionally, a selectable marker may be present in the nucleic acid construct. As used herein, the term "marker" refers to a gene encoding a trait or a phenotype which permits the selection of, or the screening for, a host cell containing the marker. A variety of selectable marker genes are available for use in the transformation of fungi. Suitable markers include auxotrophic marker genes involved in amino acid or nucleotide metabolism, such as e.g. genes encoding ornithine-transcarbamylases (argB), orotidine-5'-decaboxylases (pyrG, URA3) or glutamine-amido-transferase indoleglycerol-phosphatesynthase phosphoribosyl-anthranilate isomerases (trpC), or involved in carbon or nitrogen metabolism, such e.g. niaD or facA, and antibiotic resistance markers such as genes providing resistance against phleomycin, bleomycin or neomycin (G418). Preferably, bidirectional selection markers are used for which both a positive and a negative genetic selection is possible. Examples of such bidirectional markers are the pyrG (URA3), facA and amdS genes. Due to their bidirectionality these markers can be deleted from transformed filamentous fungus while leaving the introduced recombinant DNA molecule in place, in order to obtain fungi that do not contain selectable markers. This essence of this MARKER GENE FREE™ transformation technology is disclosed in EP-A-0 635 574, which is herein incorporated by reference. Of these selectable markers the use of dominant and bidirectional selectable markers such as acetamidase genes like the amdS genes of A. nidulans, A. niger and P. chrysogenum is most preferred. In addition to their bidirectionality these markers provide the advantage that they are dominant selectable markers that, the use of which does not require mutant (auxotrophic) strains, but which can be used directly in wild type strains.

Optional further elements that may be present in the nucleic acid constructs of the invention include, but are not limited to, one or more leader sequences, enhancers, integration factors, and/or reporter genes, intron sequences, centromers, telomers and/or matrix attachment (MAR) sequences.

The nucleic acid constructs of the invention may further comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Alternatively the nucleic acid construct may comprise sequences for integration, preferably by homologous recombination (see e.g. WO98/46772). Such sequences may thus be sequences homologous to the target site for integration in the host cell's genome. The nucleic acid constructs of the invention can be provided in a manner known per se, which generally involves techniques such as restricting and linking nucleic acids/nucleic acid sequences, for which reference is made to the standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987).

In a further aspect the invention relates to fermentation processes in which the transformed host cells of the invention are used for the production of biological compounds, such as polypeptides or metabolites. A preferred fermentation process is an aerobic or micro-aerobic fermentation process. The fermentation process may either be a submerged or a solid state fermentation process.

In a solid state fermentation process (sometimes referred to as semi-solid state fermentation) the transformed host cells are fermenting on a solid medium that provides anchorage points for the fungus in the absence of any freely flowing substance. The amount of water in the solid medium can be any amount of water. For example, the solid medium could be almost dry, or it could be slushy. A person skilled in the art knows that the terms "solid state fermentation" and "semi-solid state fermentation" are interchangeable. A wide variety of solid state fermentation devices have previously been described (for review see, Larroche et al., "Special Transformation Processes Using Fungal Spores and Immobilized Cells", Adv. Biochem. Eng. Biotech., (1997), Vol 55, pp. 179; Roussos et al., "Zymotis: A large Scale Solid State Fermenter", Applied Biochemistry and Biotechnology, (1993), Vol. 42, pp. 37-52; Smits et al., "Solid-State Fermentation-A Mini Review, 1998), Agro-Food-Industry Hi-Tech, March/April, pp. 29-36). These devices fall within two categories, those categories being static systems and agitated systems. In static systems, the solid media is stationary throughout the fermentation process. Examples of static systems used for solid state fermentation include flasks, petri dishes, trays, fixed bed columns, and ovens. Agitated systems provide a means for mixing the solid media during the fermentation process. One example of an agitated system is a rotating drum (Larroche et al., supra). In a submerged fermentation process on the other hand, the transformed fungal host cells are fermenting while being submerged in a liquid medium, usually in a stirred tank fermenter as are well known in the art, although also other types of fermenters such as e.g. airlift-type fermenters may also be applied (see e.g. US 30 6,746,862).

Preferred in the invention is a submerged fermentation process, which is performed fed-batch. This means that there is a continuous input of feed containing a carbon source and/or other relevant nutrients in order to improve protein yields. The input of the feed can, for example, be at a constant rate or when the concentration of a specific substrate or fermentation parameter falls below some set point.

### EXAMPLES

A new promoter, the hemolysin promoter (P*hlyA*), was discovered from A. *niger,* which is evaluated to be useful as a very strong promoter. This promoter may be used for the overexpression of genes in *A*. *niger* and other fungi.

Analysis of EST-databases was carried out to look for relative abundance of clones of hemolysin and gpdA in Aspergillus niger. The gene which has the selected promoter in front is called An19g00210. The results are summarized in Table 1. This table shows a high EST number of the hemolysin gene.

**Table 1: Comparison of different gene ESTs of Aspergillus niger**

| Organism | Gene | Locus tag | Number of EST (100-95% identity) |
|---|---|---|---|
| *Aspergillus niger* | hlyA | An19g00210 | 312 |
| *Aspergillus niger* | glyceraldehyde-3-phosphate dehydrogenase **gpdA** | An16g01830 | 299 |
| *Aspergillus niger* | aspartic proteinase aspergillopepsin I **pepA** | An14g04710 | 23 |
| *Aspergillus niger* | glucan 1,4-alpha-glucosidase **glaA** | An03g06550 | 413 |

To analyze the existence of homologues of this gene in *Aspergillus niger* and other fungi like *A*. *oryzae, A. terreus* and *Trichoderma, ,* BLAST analysis of fungal gene databases was carried out. The results are summarized in Table 2. This analysis showed that there is another hemolysin-like gene in *A*. *niger* (An01g09980). This gene is highly homologous to the published hemolysin gene of *Aspergillus fumigatus* (Ebina et al 1994). The A.fumigatus gene has only limited homology to An19g00210 described in Table 1. There are three hemolysin-like genes in *A*. *oryzae* and one in *A*. *terreus.* For *T. reesei* no gene was found, only an unannotated putative gene sequence which has low similarity to the An19g00210. As shown in Table 2 the number of EST clones present in the database was clearly the highest for An19g00210.

**Table 2: Analysis of the databases for the presence of hemolysin genes in different fungi.**

| **Organism** | **gene** | **Identity** | **EST** |
|---|---|---|---|
| *A. niger* | An19g00210 | 100 | 193 |
| | An01g09980 | 26 | 10 |
| *A. oryzae* | A0090010000018 | 73 | 1 |
| | A0090701000257 | 38 | - |
| | A0090023000032 | 29 | 2 |
| *A. nidulans* | AN1553.2 | 71 | ? |
| *A. terreus* | ATEG_03556 | 55 | - |
| *P.chrysogenum* | Pc12g15350 | 71 | - |
| *A. flavus* | AFLA_115930 | 73 | 2 |

Based on the sequence identity level four of the abovementioned hemolysin genes are putatively orthologous to An19g00210, namely A.oryzae A0090010000018, A. terreus ATEG_03556, hypothetical protein AN1553.2 [Aspergillus nidulans FGSC A4, |CAP81162.1| and Pc12g15350 [Penicillium chrysogenum Wisconsin 54-1255] as is also shown in the phylogenetic tree (Figure 1).

By making a "Clustal W Method" Alignment (*DNASTAR, LASERGENE, MegAlign 7.0.0 (2006)) with the promoter regions of these genes two regions of homology in the promoter sequences of about 13-18bp were identified (Figure 2). One is located around -400 from ATG (for ATEG_03556 around -370) and the other one around -300 from ATG (for ATEG_03556 at around -275).

A quantitative analysis of the hlyA promoter was carried out to compare this promoter with the wild type *gpdA* promoter. The *gpdA* promoter of *A*. *niger* is known to be a very strong promoter. The mini-promoter (pAN5mini2.0 (van den Brink et al, 2000) was also used in this experiment.

The promoters were cloned in front of the *lacZ* gene of *E*. *coli* in a vector containing also a "site-directing" pyrG transformation marker (van Gorcom et al., 1988, Nucl. Acids Res. 16: 9052; Gouka et al., 1995, Curr Genet. 27(6):536-540). After transformation of *A*. *niger* with this vector, the expression cassette will integrate in the genome at a fixed position (pyrG locus), making quantitative comparison possible. The *lacZ* gene is used as reporter gene. Correct integration of the expression cassette in the transformants was confirmed with Southern blot.

The following transformants showed the correct genotype and were used for quantitative analysis.

### Promoter present in front of lacZ gene

| | | | |
|---|---|---|---|
| • | AB1.13 pAN5mini2.0 #7, #8 | | mini promoter |
| • | AB1.13 pAB94 53 #6, #7 | wildtype *A*. *niger gpdA* promoter | |
| • | AB1.13 hlyA lacZ #2, #17 | *A. niger hlyA* promoter | |

The transformants were grown in cultures for two days in minimal media with 1% glucose as C-source. After two days mycelium was harvested, mycelia extracts were made and the β-galactosidase activity and the total protein concentration were measured (Table 3).

**Table 3: Quantitative expression analysis of different promoters using β-galactosidase activity.**

| | | **specific activity** | | **Protein conc.** |
|---|---|---|---|---|
| **sample** | **extinction** | 10³[U/mg ] | % | [mg/ml] |
| pAN5mini 2.0 #2 | 0.37 | 0.2 | 4% | 0.8 |
| pAN5mini 2.0 #17 | 0.35 | 0.2 | 5% | 0.7 |
| PhlyA lacZ #7 | 28.2 | 24 | 474% | 0.6 |
| PhlyA lacZ #8 | 23.9 | 13 | 266% | 0.9 |
| pAB 94-53 #7 | 8.4 | 5 | 100% | 0.8 |
| AB1-13 #22 | 0.0 | 0 | 0% | 0.7 |

*A. niger* transformants with fusion construct of different promoters to lacZ were grown in minimal medium with glucose. In mycelial extracts ß-galactosidase activity (extinction) and the protein concentration was measured. The specific activity is calculated from these values.

These results demonstrate that the *hemolysin* promoter is a very strong promoter. Furthermore the additional box in the *gpdA* promoter has a positive effect on expression. The gpdbox promoter results in higher expression than the wildtype *gpdA* promoter which is present in the pAB94-53.

To analyze the utility of this promoter on other C-sources than glucose, this experiment was repeated with different C-sources (maltose, xylan and xylose). The results are shown in Table 4.

**Table 4: Quantitative expression analysis of gpdA and hlyA promoter on different C-sources, using β-galactosidase activity.**

| | | **Specifc activity** | | **Protein conc.** |
|---|---|---|---|---|
| **C-source** | **transformant** | **U/mg (* 10³)** | % | **Mg/ml** |
| glucose | PhlyAe lacZ | 13.4 | 330 | 0.35 |
| glucose | pAB94-53 | 4.1 | 100 | 0.68 |
| glucose | Parent strain | 0 | 0 | 0.60 |
| maltose | PhlyAe lacZ | 11.6 | 200 | 0.88 |
| maltose | pAB94-53 | 5.9 | 100 | 1.30 |
| maltose | pAN5mini | 0 | 0 | 1.68 |
| xylan | PhlyAe lacZ | 9.6 | 240 | 0.68 |
| xylan | pAB94-53 | 4.0 | 100 | 0.92 |
| xylan | pAN5mini | 0 | 0 | 0.96 |
| xylose | PhlyA lacZ | 8.9 | 205 | 0.49 |
| xylose | pAB94-53 | 4.3 | 100 | 0.86 |
| xylose | pAN5mini | 0 | 0 | 0.74 |

*A. niger* transformants with fusion constructs of different promoters to lacZ were grown in shake flask cultures with minimal medium with glucose, maltose, xylan or xylose as C-source. In mycelial extracts ß-galactosidase activity (extinction) and the protein concentration was measured. The specific activity was calculated from these values

These results show that the *hemolysin* promoter is highly expressed on different C-sources like glucose, xylose, xylan and maltose.

AB1-13 PhemolacZ#7 was grown in a controlled batch fermentation experiment on Minimal Medium sparged with 25%air/75%nitrogen. Mycelial extracts were made from samples isolated during fermentation and the ß-galactosidase activity and protein concentration from these extracts were measured. The results are shown in Table 5. High ß-galactosidase activity could be determined in mycelial extracts of AB1-13 PhemolacZ#7 until the end of fermentation (see table 5).

**Table 5 Transformant AB1-13 PhemolacZ#7 was grown in controlled batch fermentation under oxygen limitation (25%air/75%nitrogen) on MM with glucose. In mycelial extracts, ß-galactosidase activity (extinction) and the protein concentration was measured. The specific activity is calculated from these values**

| | Fermentation time (hours) | Specific activity (* 10³) U/mg | Protein conc mg/ml |
|---|---|---|---|
| P hemo-1-2 | 24 | 3.5 | 4.5 |
| P hemo-2-2 | 42 | 16.2 | 3.1 |
| P hemo-3-2 | 48 | 16.3 | 3.2 |
| P hemo-4-2 | 66 | 24.1 | 2.6 |
| P hemo-5-2 | 72 | 23.7 | 2.6 |
| P hemo-6-2 | 92 | 24.3 | 2.3 |
| P hemo-7-2 | 96 | 33.6 | 2.2 |
| P hemo-8-2 | 162 | 34.6 | 2.1 |
| | | | |

Our results show that the *PhlyA* promoter is a really strong promoter, even better then the currently widely used *gpdA* promoter. In addition our results show that the *PhlyA* promoter is a constitutive promoter, as similar specific activity was observed on different carbon sources and in controlled batch fermentation

### SEQUENCES

SEQ ID NO: 1 hemolysin promoter sequence from A. niger, the actual
promoter sequence runs up to the ATG start codon (shown in bold, underlined)
at position 1131
1 GCGGCCGCGT CTGTCAAGTA AGTTATGTGT AGTGCATCCC TAGCTTTCGG CCAATTCCTG
61 CCTGCATTGG TGCGCCGGCC TCCTACTACG CAACCGGACG AGTCTACTCC CTACCATGTT
121 GGAGTTCATT GACCCTCAAA AGGCATCAAA CTGAAAGGAC CATGCTAGCA GTAGAGAGAG
181 TAAGGAGCCA TTCTATTTCA GATCAGCAGC ACTTCTGGGT AAAATGCAAG CCATTATTCG
241 TTAGCTGTAA CCAAGATTGA TCCCCGACAT TTATCTGGCC CTCCTATCAG CACTGGCCAC
301 GAGTCCTGCA TGATGGTACG AAGCTGCAGG CATTACGTCC AACAAAGTGG TTTCGAGAAA
361 TTCCAGTGGT TCAAAAGCCA GGTACTTTTG GATTTGAGCC AATAGACGAA ACCGGGCTTA
421 TTGGTACCAT CACGAGGGGA AGATTTAGGT GGAAACCGCC AACTTGAAAC CTTTTGGCCA
481 GGTCTCTCTG GCCAAATTCC CAGACCAGCA GTCTCGCCAC CAAGGGAAAT AGGTGACATG
541 TCATCCAATT AGAAAGATGG AGCAGCATCG ACAGCTCTGC CGATCCCTGG GACAACAACT
601 GGTGATCATT GGGCGGGTGG TGCGGGGAAA ACAAGCCGGG GGTAACAGAC GAAGCCAAAG
661 CAGCAACACC AAAAAAGTGG TGATGCCATC GGTGCCGGCG ACAAAGATAC CACGGGCCAT
721 GACGCCACCA GTAGCAACGC CATGCGAGCA ACGAAACACA ATAGAGCCAG TGAGCAGCAG
781 CCCGTCAACT GCCGGCCCAC CCCCACAATC CCAGCATCTG CATGTCTCCA TCCGTGGATC
841 AGCCCACCGC CGAGCCAACC ACATCAACCC ACCCTGCCGT CATCTGATCC TCTTTCCTCA
901 CTGTCTCATC TCTGGCTCCT CTCCGTCCAT TCATCCAAGC AAATTGGGAG ACTGATCAGC
961 CCTTGTCCAA TGCCGCTCTC GAATCACCCC ACAGCCCGGC CTCAGCAATA AATAGCCCCT
1021 TCGGTAGCTG CTCGACTCGC TCTTCTCTCT CCCATCATCC TCAAAGCATC ACAGCATACA
1081 TAGATACACA CATACCCTTT CTACTCACCT TCTGCCCTGC TTCATTTACC ATGGCC

SEQ ID No 2: hemolysin promoter sequence from A.oryzae AO090010000018,
the actual promoter sequence runs up to the ATG start codon (shown in bold,
underlined) at position 1101

1 CTGCTGGTGC CCACGGGATC GTAAATGGAC CTTGACCAAT TCGAGTAGTT CCGGAGACGA
61 TCGCTCATCG CTCGATTTGG TCTCGTGTCC AGAGTGTTGC TACAGCATGG TCTGGATTCC
121 AATCCACGCA GCAGATATCT CTTTTACGCA GTAACTATTC GATAGCCATT TCCGTTCAGA
181 TCAGCCGTCG GATCCGAGGA GCGACGACAT CAATGCGTGT TATTAGTCAA ATTTTGGAGG
241 GGTTGCGTGC CCACTGCAGG CAGATGTAGC CGTGGCACCA CAACACCGGC CAGCCCTGGA
301 TTGGGTGGTG GAACCAAGAT ATGAGAACCA GTATCTATTG GCACGGAGGC GTTTCCGGAG
361 CCTGCCGCGT GTGATACGTG CAGAGTCAAT TCCTACGGAC TGTCTGGGGT AGGATCACAA
421 CTAATCAGTT GCAGCGATGG TTTGACAACA GGGGTCGATC AAAGTTTCCG AAGAATAGGA
481 AGCGAGGACA GCACCAAGGC CGCTGAACCA CAGGAAACAA AGGAAGGAAA AACACAACAA
541 AACCAAAGAC AGACACATAG GAAATGACAT ACTTACCAGA GATAAGATGA AAAGCACCAT
601 GGGGAGGGAG GGTATGGATG GGGAAGTTGA TCAACAGTCT GAAACCCGCC CGAAATGAAT
661 GCATGACGCG ACGATTCCAT CTCCACCTAA GCTTCATCCC GTCAACCTCT AACAACGCGC
721 TCGGAGGAGA AAAAGAGGGG GATCAACAGC AATCTAGGTT GTTGTTCCCC CAGGATTTCT
781 CCGCAAAGAA TGTTTAGGGT TCCGTGGATC GGGTGACCGA ATCGGCCAAC CGCATTGTCT
841 GATCGTCTCG TCATATGATC CAGTGCATGA CGTCTTCCAT CAATCCATCA CCCCAGACTT
901 GAATCCTCAC ATTAATTCAA CAATTGTCGC TCGGGGAAAT CAATAAATAC CCGCTCGTCT
961 CCTCCCTCCC TCGTGCTGGT TCAGTTGATT GTTCACTCAT CGACTTTATC AATCTTCCAT
1021 TGACCATTCC AGGCTTGTCG CCCACTCATA TAATCTTCTT TCCCCGGTCT CACATCAATC
1081 ACCCTTCACA CCACAACACC ATG

SEQ ID No 3: hemolysin promoter sequence from P. chrysogenum Pc12g15350,
the actual promoter sequence runs up to the ATG start codon (shown in bold,
underlined) at position 1194

1 AATGAGACTG GAGGAGCGAA TGTGGGATTA TAGCTTGAAG CAGAGCCGCG TTTGTAGATC
61 GCCCAGCACT GCCTCTGGCC CCAGGGCCCC ATGGCCCCCT TGTTGTTTCA CCCACTTTCG
121 CATAACTAAA AGTGTCTATT AATAATAGCT TTTGACTATC TAAAGGAAAT TTGCAAATAC
181 TCATCGGGAA TTGAAAAACA GCCTTGCCGT TCAGGCTATT CACGGCCCGC CTGCCCGCCC
241 CCGGATTTCC TGGTTGGCAT ACATTGCCAC GGGAATTGAT AATATAGCTT CAGGATATTT
301 GGACGTAGTT GATAATATAT TGAAGCCGGG CACTTCCACT CAAATGGGAC GCCATTGAGC
361 TCCAATGCTC ACATGGACTG CCTTATAGCC CTGTTGCCAG AACCGCCCGT CATGAGTAAG
421 ACGGGCTTGA AGTGTCCACA TAGGTACAGG TCAGTTTAGG TGTCTTGATC AATATGCTTG
481 GTGTATGACA CGACAATGAA CACCCTCGAA GCACAAGTCT ATGTGACCCA AATGTCGTTA
541 ATGCAATTAT GCTGTATGGA GATTGGGGGA TTGGGGGATT GGGGGATTGA ACCCATTGGC
601 GGTCATGTTT CTCTGAGTAC TTTTTAAGTC AATTTGGTGG AATAGACAAA GGCTGTCCTC
661 ATGCAACAAG TCACTCGGCA TCTTGTCCCT TGGTCTGTCT CAAATTATCG TGTTTCACTC
721 CAGTTGAGTT TGTATTTTGT ATACCTGGCA GAGTCAGTGG TTCCATCCTT CTTATTTTGT
781 TATAGTCTTA TCTTGCCATC CTTATTGCCC TAGAGCTGTT TAATGTACGG GATCTCCGGT
841 CAACTTCACA TAATGTCATG CGTTCCATTG CCCAATTATC ATGCCACTCC GCACTACTCA
901 GATCACGCGG TCTCAAATGA TGCAATAAAA CAAGGTGATA GGCAGGTGAA CCCCGCAAAT
961 ACCAGCAAGG TTCTCTTGTC CAGGTCGGTC ATTGGACAAT GGTGTTGACG GTCGTGCCAT
1021 TCGTCGCCTA CCCGTCGACC CGTGGACGTA TTCAGAATGA ATTACATCAC CACAGGCGCT
1081 CCCCCGCATT CTGGGGTTTG ATGCATATAA AGAGTTGACA TTTCTGAAAA GGAGTTTGAT
1141 TTCTACAATT CTTCGATTAT CACTTATCAC AATTACTTCA CAGATTTTCC AATATG

SEQ ID No 4: hemolysin promoter sequence from A. terreus ATEG_03556, the
actual promoter sequence runs up to the ATG start codon (shown in bold,
underlined) at position 1097

1 GGTGTCGGAG GATAAGAGTG GTAATAAGGT CTTTCTAGGA TCTTGATTGG AATGTCTGCT
61 AGAGATGAAG ACTGATCAGG TACTTCTGCG GGTTATAAAG ACGACCTCCG CCTGCACCAT
121 TCCATCCTCA CTGCGGGGAA ACTATCACCT GATGGACTCT CGCATGGGGT GATGAGGTCA
181 TGATGATCGC CATGAGACAC GTCGCCTTTG TTGTGTGAAA GGGCATTCTG CGGAGGGGAG
241 GCGAAGCGGG TGGTGAGATC ATGAAGGCGG ATGTGCTGGT CTGATAGGGT GGAGGATCAT
301 TGTTGCGGAA GTCAATGGCA TGATGGACCC TCTTGGATCC GGAGTTCCCC AACATCCAGC
361 CCGCTGGAAG GCCCCCGAGA TGCGGGGAAA TTACGACCAC TGCCTGGGTA GATGCTGATC
421 GTTGGGACGG AGGGACACTG GTAGTTGGAA AGGGATGATG GTGATGATGG CAAGGGCGAC
481 TTCATTATGC TTCACTCTGG ACGAGGACTG CAACACCCGA GCATCCATCC ATATGGTCAC
541 GAGACCAGCC TCGTATGATT CGGATGATGG GGAATAGCCT CAATATCATG ATCGCACGGA
601 TAAAAATAGT CTAAAATATC GTCTAATGCT GGTGTGATGC TCAAGTCGTT CCACCCATCT
661 ATTCCCTATT CGCAGCATGC TTGCTGAACT TCCGTATGCT TGGTCCATAA ATATAAAGCA
721 TGTACGGAAT CACCGCCATC ACCGCACTAA TCGCTCCCAG AATCGTCAGA GTATACTGGA
781 CACCGATGGA CCGATATATG GGAGCTCCGG CGACCGTGAT GCCTCCCGCG ACCAGGTACC
841 GCGAGAAGAC GACAAACCCC AGCGCGGACG CTGAGTAAAC CCCATAGGAG TCGATGATGT
901 ACATATAGGC GACGATGAAG ATCGTGGTGG TGCCAAATCC AAATAGGACA GAGGCAACCA
961 GAGGCGACCA GATGGAAATG TTGGGCTAAA CAAGAAGGTC AGTTCTCGAT CGTTGGGTCT
1021 GCGAGAGATC ACACTTACAT AGCTCGTCCA ACCCATCCAG AACAGGCTGA CTGGAACCGC
1081 AGGGGCGCCT CCCAACATG

SEQ ID No 5: hemolysin promoter sequence from A. nidulans AN1553.2, the
actual promoter sequence runs up to the ATG start codon (shown in bold,
underlined) at position 1272

1 CGTACGAATG GGCCAAGCAG GCTGCTGAGC TCGGTGAGTT GTCTGACACT GTTTATAATA
61 ATTCCGGCTA ATGTAAAACA GGTCTTGCAA AAGCACAATA TGCTGTAGGT TACTTCACGG
121 AGATGGGTAT TGGATGTCGA CGTGATCCCT TGGAGGCCAA CGTCTGGTAC GTCAAAGCGG
181 CCGACCAGGG AGATACCAGA GCGGTGCACC GGATCGCAGC AATTCGAGCT GCGGCGGACG
241 GTGTCAACCC AGCGCAGGCC GCTGCGGATG CCAACGGGAA GAAGCGTAAA AATGGTACGT
301 CTCCAGTTTT GACTTATTCT ACTAATTCTA ATTGATGAAT GATCCAGATG GCAAGAAAAG
361 ATTCGGTATT TTCTAAGCCT ACCATGACAT TAGCAATTTA CACAGCGGAC ACAAGCGACA
421 TAGTCCGCGA TGGTTAATAC ATAATACACA CGATTTGCAC ATGTACGGGC ATTGGTACTC
481 TTATCTTACT GCAGCGAGTT GCTATGCATG GATTTGTTTG GCCGCATTTG GGAGGGCATC
541 CGGACCATGT TTATTTTTAC CAGACGAACA TATCCTCTGC ACATAGTTCG ATTTGATATC
601 TCCTCTTCTG TGCGATTACC ATCGTACAAA TAGTACACAT CTTTCGAGGG TTCAACCAGC
661 TGAAACCCTG ATCTTCCCTA GAATTATCCT TGGATGTAAC AAAGGCCTGC TGTTGGCTGT
721 CAGGTATAGC AGTCGTCATC ATGGCAGCTT TCCCCGCATT CTCTCCGCCT CCGTCCATTC
781 TCCAGATTCC ATTGCTCGCT ACCATTGGAG ACGGTCCTAT CACCCAAACT ACCCGACCTA
841 TCAAACACAC ACTCCCTTAC CGAAGGTTCA TCATGACTTC ATCACATTCG AAGAGCCAAC
901 AGATGCCCAT CCACAGGTCT CCGCCATTCA GCATGCAATA CCCCGCAGGA AGCATATAAA
961 CGCACCCTGA CCTCCCCTCC TACTCAATTA TATCAAGCTA CTGAATACCT ATTCTCGCAA
1021 CACATACAAG CTCAATTCAT TCAAGATGGA ACCGGCAGAC CCCCTTGACG GCCAATGGAT
1081 CTCCATCCAC ATTCGTGACC GCCTCAAAGA CCGTGACATC AGGATCCGCG ACTCCCTAAT
1141 TACTCCGTCT GTCTCCATCT TTCCCACCTA CATTCTATCT CAGCCTCACT AACATGACAT
1201 AAACCCGCAG TGGCGACTTC CACTCGCCGG ACAACCCTGC AGAGCCGCTC ACAGAATCCG
1261 CTATTGACAA AATG

## Claims

1. Promoter derived from the *Aspergillus niger* hemolysin gene, comprising the sequence 1-1130 of the sequence depicted in SEQ ID NO: 1 or a truncated variant thereof that has promoter activity, or a sequence that is more than 70% identical therewith, preferably more than 80% identical therewith, more preferably more than 85%, more preferably more than 90%, more preferably more than 95%, more preferably more than 96%, 97%, 98%, or 99% identical therewith.

2. Promoter according to claim 1, **characterized in that** it is chosen from the group of orthologous promoters, comprising the promoter of the gene encoded by A0090010000018 from A. oryzae (SEQ ID NO:2), the promoter of the gene encoded by Pc12g15350 from *P. chrysogenum* (SEQ ID NO:3), the promoter of the gene encoded by ATEG_03556 from A. *terreus* (SEQ ID NO:4) and the promoter of the gene encoded by AN1553.2 from *A*. *nidulans* (SEQ ID NO:5).

3. A DNA construct comprising a promoter DNA sequence according to claim 1 or 2 and a coding sequence in operative association with said promoter DNA sequence such that the coding sequence can be expressed under the control of the promoter DNA sequence.

4. A host cell, preferably a fungal host cell, comprising the DNA construct according to claim 3.

5. The host cell according to claim 4, wherein the host cell is a cell from the genus Aspergillus, Penicillium, Chrysosporium, Neurospora or Trichoderma.

6. The host cell according to claim 5, wherein the host cell is an *Aspergillus niger, Aspergillus terreus, Aspergillus oryzae, Aspergillus nidulans, Chrysosporium lucknowenses, Trichoderma reesei,* or *Penicillium chrysogenum* species.

7. A method for expression of a coding sequence in a suitable host cell comprising:
(a) providing a DNA construct according to claim 3,
(b) transforming a suitable host cell with said DNA construct, and
(c) culturing the suitable host cell under culture conditions conducive to expression of the coding sequence.

8. A method for the production of a biological compound in a suitable host cell comprising:
(a) providing a DNA construct as defined in claim 3,
(b) transforming a suitable host cell with said DNA construct, and
(c) culturing the suitable host cell under culture conditions conducive to expression of the coding sequence, and optionally
(d) recovering the biological compound from the culture broth.

9. A method according to claim 8, wherein the biological compound produced is a polypeptide.

10. A method according to claim 9, wherein the polypeptide produced is encoded by the coding sequence present in the DNA construct as defined in claim 3.

11. A method according to claim 8, wherein the biological compound produced is a metabolite.

12. A method according to claim 11, wherein the coding sequence present in the DNA construct as defined in claim 3 encodes an enzyme involved in the production of the metabolite.

13. Use of a promoter according to claim 1 or 2 for the production of a biological compound in a suitable host cell.
